# EUROPEAN PATENT SPECIFICATION

(11) **EP 0 362 706 B1**
(45) Date of publication and mention of the grant of the patent: **17.01.1996**
(21) Application number: 89118040.8
(22) Date of filing: 29.09.1989
(51) Int. Cl.: C07K 5/06, A23L 1/236

(54) **Process for preparing dry IB type crystals of alpha-L-aspartyl-L-phenylalanine methyl ester having improved solubility**
Verfahren zur Herstellung von trockenen IB Kristallen des alpha-L-Aspartyl-L-phenylalaninmethylesters, die eine verbesserte Löslichkeit besitzen
Procédé pour la préparation de cristaux IB secs de l'ester méthylique de l'alpha-L-aspartyl-L-phénylalanine ayant une solution modifiée

(30) Priority: 03.10.1988 JP 249682/88
(43) Date of publication of application: 11.04.1990
(60) Divisional application: 95109112.3
(73) Proprietor: AJINOMOTO CO., INC., Tokyo 104 (JP)
(72) Inventor: Kishimoto, Shinichi c/o Central Research Lab., Kawasaki-ku Kawasaki-shi Kanagawa-ken (JP); Naruse, Masayoshi c/o Central Research Lab., Kawasaki-ku Kawasaki-shi Kanagawa-ken (JP); Ohura, Harutoshi c/o Tokai Plant, Yokkaichi-shi Mie-ken (JP); Yasaki, Akihiko c/o Central Research Lab., Kawasaki-ku Kawasaki-shi Kanagawa-ken (JP)
(74) Representative: Strehl Schübel-Hopf Groening & Partner

(56) References cited:
- EP-A- 0 091 787
- EP-A- 0 119 837
- EP-A- 0 255 092
- EP-A- 0 256 513

## Description

The present invention relates to a process for preparing dry I_{B} type crystals of α-L-aspartyl-L-phenylalanine methyl ester (hereafter simply referred to as aspartame) having improved solubility.

Aspartame is a refreshing sweetener which has a minimized bitter taste or aftertaste generally involved in a highly sweet sweetener. It has thus found widely spread use as a low calorie sweetener. In terms of physical properties, however, it is disadvantageous that aspartame has a poor dispersibility and solubility in water.

In order to improve the dispersibility and solubility of aspartame several approaches have been made to modify aspartame preparations, for example by forming granules, preparing effervesent powders or tablets and the like by adding excipients and disintegrators to the preparations. The co-presence of additional substances, such as excipients, often causes trouble depending on the final use of the aspartame. It is therefore a problem to be solved to provide aspartame having high purity, but additionally good solubility and dispersibility in water and other liquids.

In an attempt to improve the solubility of aspartame while maintaining the purity, there has been developed a method which comprises spray-drying aspartame in its slurry state (Japanese Patent Publication No. 58-20588), a method which comprises adding water to aspartame to a specific water content and granulating the aspartame (Japanese Patent Application Laid-Open No. 59.95862).

EP-A-119837 (JP-A-59-172444) relates to aspartame II crystals having good storage stability and good solubility, which are produced by drying conventional aspartame I crystals at a temperature in the range of about 80°C to 150°C. This document therefore relates to a conversion of I type crystals to II type crystals in the drying step. Another process for producing dry aspartame having improved solubility is disclosed in EP-A-0255092, where a two step drying process at very low temperatures is described. As stated in this document, the use of higher drying temperatures will result in the production of the undesired II type crystals and diketopiperazine.

EP-A-0256513 discloses a method for producing dry aspartame having improved solubility by drying wet crystals of aspartame, according to which it is essential that the wet aspartame is granulated to have a specific surface area of at least 4 m/g before the drying step which is preferably carried out at temperatures lower than 80°C.

Although it is desirable to prepare pure I_{B} type crystals in an industrial scale by an efficient and simple method, the prior methods are not yet satisfactory, since they either result in a mixture of I type and II type crystals or they require too many process steps and a low drying temperature.

If it is attempted to dry wet crystals of aspartame at a low temperature below 80°C, a long period of time is required. Such a procedure is not an efficient drying method from the industrial viewpoint. Further in order to obtain I_{B} type crystals in high purity, complicated operation control is also required. From technical and economical viewpoints, it is thus an important problem to develop a process for continuously preparing I_{B} type crystals having excellent solubility in a short period of time.

As a result of extensive investigations in order to solve the foregoing problem, the inventors have developed a process for preparing dry I_{B} type crystals of α-L-aspartyl-L-phenylalanine methyl ester having improved solubility, which comprises drying wet crystals of α-L-aspartyl-L-phenylalanine methyl ester. This process is characterized in that
(a) the crystallization of α-L-aspartyl-L-phenylalanine methyl ester is carried out without causing a forced liquid flow in the crystallization medium,
(b) the formed crystals are separated from the crystallization medium, and
(c) wet crystals having a water content of not more than 50 wt.%, based on the overall weight of the wet crystals of α-L-aspartyl-L-phenylalanine methyl ester, are continuously air dried by a pneumatic conveyor dryer with hot air at 80 to 200° C until the water content has reached 2 to 6 wt.%.

According to the present invention, it is possible to control the water content of the formed aspartame crystals to a low water content by applying non-stirring crystallization conditions. By drying the obtained wet crystals with hot air at 80 to 200° C while supplying and preferably recycling the hot air, aspartame in the form of I_{B} type crystals having good solubility can be readily prepared on an industrial scale.

In order to obtain the aspartame wet crystals by crystallization without stirring according to the present invention, one may use the method for crystallization disclosed in EP-A-091787 (Japanese Patent Application JP-A-58-177952). More specifically, the method comprises a step of cooling by conductive heat transfer without applying forced flow, such as mechanical stirring etc., in the crystallization of aspartame from an aqueous solution of aspartame in such a way that the amount of the crystallized solid phase is approximately 10 g or more per liter of solvent after the cooling. By this method a pseudo solid phase is formed. After the pseudo solid phase has been formed, the system is subjected to solid-liquid separation as it is; or if necessary, after further cooling, the solid-liquid separation is performed. Thus, the aspartame wet crystals can be obtained through crystallization without stirring.

The aspartame crystals obtained by the non-stirring crystallization are composed of bundles of fine needles, that is bundle-like crystals apparently forming one crystal mass and which show very good dehydration property in the solid-liquid separation.

If, on the other hand, the crystallization of aspartame from an aqueous solution is carried out by cooling with stirring, that is in a process which comprises a step of performing forced flow such as by mechanical stirring, which has been the usual industrial method, it has been found that a slurry-like mixture of mother liquor and crystals is formed, in which the obtained aspartame crystals are needles which after filtration still have a high water content of approximately 75 %. Even after centrifugation the water content is slightly over 50 %. Therefore, when the wet aspartame crystals obtained by crystallization under stirring are fed to a dryer, such as a jet air dryer, it is unavoidable that the wet crystals will adhere to the inner wall of the dryer resulting in overdrying and scorching of the adhering crystals. This process therefore results not only in procedural difficulties, such as breakdown by clogging of the apparatus, but also to an inferior quality of the obtained product. Since furthermore the dispersibility of the obtained crystals in the air flow is poor in the drying process, the product shows the tendency of forming large agglomerated masses. Furthermore, in the step of feeding the wet crystals to the dryer, the crystals tend to adhere to the feeding screw, already leading to difficulties during the feeding step.

To the contrary, the aspartame wet crystals provided by crystallization without stirring can be fed to an air flow drier after the centrifugation as they are, since the water content is generally 20 to 50 % and in the case of a further centrifugation, approximately 10 to 20 %. Even in this case, the foregoing problems are not caused but the drying can be performed smoothly and efficiently to prepare the I_{B} type crystals of aspartame in high purity. The aspartame wet crystals also include granules in a wet crystalline state, which are obtained by extruding the wet crystals through a screen.

Suitable devices for continuously drying while flowing air in the process of the present invention are conventional dryers used for continuous air drying, known as pneumatic conveyor dryers, which are described e.g. in Perry's Chemical Engineer's Handbook, 6th Edition, 1984, McGraw-Hill, pages 20-51 to 20-54.

When aspartame is dried at a high speed, a part of aspartame tends to be converted into diketopiperazine derivatives which are not sweet at all, resulting in a loss of the overall sweetness. According to the process of the present invention, the drying is continuously carried out while flowing hot air and completed in a period as short as within about one minute. Even when using hot air at high temperatures of 80 to 200° C, desirably 130 to 160° C, the short drying period suppresses the conversion to diketopiperazine derivatives, and the formation of type II crystals described in EPA-119837 (Japanese Patent Application Laid-Open no. 59-172444) is minimized. Accordingly, I_{B} type crystals of aspartame can be prepared stably with extremely good efficiency. In case that the temperature of hot air is below 80°C, a long period of time is required for the drying. Conversely, when the temperature is higher than 200°C, the rate of causing rearrangement to type II crystals increases. Therefore, temperatures outside the above range are not preferred for industrially preparing I_{B}, type crystals stably.

Dry crystals (I_{B} type crystals) of aspartame having a water content of approximately 2 to 6% can be obtained by the continuous drying while supplying hot air. The obtained I_{B} type crystals of aspartame have good solubility and show excellent powder properties, such as minimized scattering properties in handling.

The present invention is described in more detail by referring to the examples below.

### Example 1

A raw aqueous solution, 380 liters, in which 17.7 kg of aspartame (55° C, initial aspartame concentration of 4.4 wt%) had been dissolved was charged to a crystallization device having a diameter of 400 mm equipped with a jacket and having a cooling plate in the inside. A coolant having a temperature of 0° C was cycled through the jacket and the cooling plate to cool the solution for over 3 hours. About an hour after, the solution as a whole became a pseudo solid phase. The aspartame crystals in the pseudo solid phase were brought into a receiving tank equipped with a stirrer and homogenized to form a slurry. The slurry was further cooled (from 16°C to 7°C) in the receiving tank. The thus obtained slurry was filtered and dehydrated with a centrifuging device having a diameter of 91.5 cm (36 inches) to give aspartame wet crystals having a water content of 30 %. The wet crystals obtained by such non-stirring crystallization were continuously fed to a Micron drier (manufactured by Hosokawa Micron Co., Ltd.) using a screw feeder.

### Drying conditions:

| | |
|---|---|
| Temperature of hot air at drier inlet | 135°C |
| Temperature of waste air at drier outlet | 104°C |

The amount of the I_{B} type crystals after drying was determined by the ratio of characteristic peaks of the I_{B} type crystals to II type crystals in powdery X ray diffraction patterns (the same also applies to the following examples).

### Results:

| | |
|---|---|
| Water content | 2.6% |
| Amount of I_{B} type crystals | 95% |
| Diketopiperazine derivatives | less than 0.05 % |

### Example 2

Aspartame wet crystals (water content of 30% based on the weight of aspartame wet crystals) were obtained by non-stirring crystallization in a manner similar to Example 1. The wet crystals were fed to the same Micron drier as in Example 1 and air dried under drying conditions described below.

### Drying conditions:

| | |
|---|---|
| Temperature of hot air at drier inlet | 160°C |
| Temperature of waste air at drier outlet | 109°C |

### Results:

| | |
|---|---|
| Water content | 2.4% |
| Amount of I_{B} type crystals | 87% |
| Diketopiperazine derivatives | less than 0.05 |

## Claims

1. A process for preparing dry I_{B} type crystals of α-L-aspartyl-L-phenylalanine methyl ester having improved solubility, which comprises drying wet crystals of α-L-aspartyl-L-phenylalanine methyl ester, characterized in that
(a) the crystallization of α-L-aspartyl-L-phenylalanine methyl ester is carried out without causing a forced liquid flow in the crystallization medium,
(b) the formed crystals are separated from the crystallization medium and subjected to a dewatering treatment to obtain wet crystals having a water content of not more than 50 wt.%, based on the overall weight of the wet crystals of α-L-aspartyl-L-phenylalanine methyl ester, and
(c) the obtained wet crystals having a water content of not more than 50 wt.% are continuously air dried by a pneumatic conveyor dryer with hot air at 80 to 200° C until the water content has reached 2 to 6 wt.%.

2. A process according to claim 1, wherein the crystallization in step (a) is carried out without stirring the crystallization medium.

3. A process according to claims 1 or 2, wherein the crystallization in step (a) is carried out by cooling by conductive heat transfer.

4. A process according to any of the claims 1 to 3, wherein the drying temperature is 130 to 160° C.

5. A process according to any of the claims 1 to 4, wherein the wet crystals having a water content of not more than 50 wt.% are obtained by centrifugation.

6. A process according to any of the claims 1 to 5, wherein the drying in the pneumatic conveyor dryer is carried out at an inlet temperature of 80 to 200° C, preferably 130 to 160° C, and an outlet temperature of 50 to 180° C, preferably 70 to 130° C.

## Patentansprüche

1. Verfahren zur Herstellung von trockenen Kristallen des I_{B}-Typs von α-L-Aspartyl-L-phenylalaninmethylester mit verbesserter Löslichkeit, welches das Trocknen von feuchten Kristallen von α-L-Aspartyl-L-phenylalaninmethylester umfaßt, dadurch gekennzeichnet, daß
(a) die Kristallisation von α-L-Aspartyl-L-phenylalaninmethylester durchgeführt wird, ohne daß eine Flüssigkeits-Zwangsströmung in dem Kristallisationsmedium verursacht wird,
(b) die gebildeten Kristalle aus dem Kristallisationsmedium abgetrennt und einer Entwässerungsbehandlung unterworfen werden, so daß feuchte Kristalle mit einem Wassergehalt von nicht mehr als 50 Gew.-%, bezogen auf das Gesamtgewicht der feuchten Kristalle von α-L-Aspartyl-L-phenylalaninmethylester, erhalten werden, und
(c) die erhaltenen feuchten Kristalle mit einem Wassergehalt von nicht mehr als 50 Gew.-% mit Hilfe eines Drucklufttrockners mit Heißluft von 80 bis 200 °C getrocknet werden, bis der Wassergehalt 2 bis 6 Gew.-% erreicht hat.

2. Verfahren nach Anspruch 1, wobei die Kristallisation in Stufe (a) ohne Rühren des Kristallisationsmediums durchgeführt wird.

3. Verfahren nach Anspruch 1 oder 2, wobei die Kristallisation in Stufe (a) unter Kühlen mittels Wärmeleitung erfolgt.

4. Verfahren nach einem der Ansprüche 1 bis 3, wobei die Trocknungstemperatur 130 bis 160°C beträgt.

5. Verfahren nach einem der Ansprüche 1 bis 4, wobei die feuchten Kristalle, die einen Wassergehalt von nicht mehr als 50 Gew.-% haben, durch Zentrifugieren erhalten werden.

6. Verfahren nach einem der Ansprüche 1 bis 5, wobei das Trocknen in dem Drucklufttrockner bei einer Eingangstemperatur von 80 bis 200°C, vorzugsweise 130 bis 160°C, und einer Austrittstemperatur von 50 bis 180°C, vorzugsweise 70 bis 130°C, durchgeführt wird.

## Revendications

1. Procédé de préparation de cristaux secs du type I_{B} d'ester méthylique d'α-L-aspartyl-L-phénylalanine ayant une solubilité améliorée, lequel comprend le séchage des cristaux mouillés d'ester méthylique d'α-L-wpanyl-L-phénylalanine, caractérisé en ce que :
(a) on met en oeuvre la cristallisation d'ester méthylique d'α-L-aspartyl-L-phénylalanine sans provoquer de circulation forcée de liquide dans le milieu de cristallisation,
(b) on sépare les cristaux formés du milieu de cristallisation et on les soumet à un traitement d'élimination d'eau pour obtenir des cristaux mouillés ayant une teneur en eau inférieure ou égale à 50 % en masse, par rapport à la masse totale des cristaux mouillés d'ester méthylique d'α-L-aspartyl-L-phénylalanine, et
(c) on sèche continuellement à l'air les cristaux mouillés obtenus, ayant une teneur en eau inférieure ou égale à 50 % en masse, par un sécheur à transporteur pneumatique avec de l'air chaud à une température comprise entre 80 et 200°C jusqu'à ce que la teneur en eau ait atteint 2 à 6 % en masse.

2. Procédé selon la revendication 1, dans lequel on met en oeuvre la cristallisation de l'étape (a) sans agiter le milieu de cristallisation.

3. Procédé selon les revendications 1 ou 2, dans lequel on met en oeuvre la cristallisation de l'étape (a) en refroidissant par transfert de chaleur par conduction.

4. Procédé selon l'une quelconque des revendications 1 à 3, dans lequel la température de séchage est comprise entre 130 et 160°C.

5. Procédé selon l'une quelconque des revendications 1 à 4, dans lequel on obtient par centrifugation les cristaux mouillés ayant une teneur en eau inférieure ou égale à 50% en masse.

6. Procédé selon l'une quelconque des revendications 1 à 5, dans lequel on met en oeuvre le séchage dans le sécheur à transporteur pneumatique à une température d'entrée comprise entre 80 et 200°C, de préférence entre 130 et 160°C, et à une température de sortie comprise enfin 50 et 180°C, de préférence entre 70 et 130°C.
